# EUROPEAN PATENT APPLICATION

(11) **EP 2 399 566 A1**
(43) Date of publication of application: **28.12.2011**
(21) Application number: 10305697.4
(22) Date of filing: 28.06.2010
(51) Int. Cl.: A61J 7/04, A61K 31/565, A61K 31/57, A61P 15/18

(54) **Once-a-month method of contraception**

(71) Applicant: Laboratoire HRA Pharma, 75003 Paris (FR)
(72) Inventor: Ulmann, André, 75005, PARIS (FR); Gainer, Erin, 75019, PARIS (FR); Levy, Delphine, 75011, PARIS (FR)
(74) Representative: Chajmowicz, Marion

(57) **Abstract**

The invention provides a method for providing contraception in a woman, wherein the woman is administered with a progestogen-only contraception composition once-a-month between the 1^{st} and the 10^{th} day of the woman menstrual cycle. It is further provided a system for reminding a woman of taking such contraceptive composition.

## Description

The present invention relates to a method for providing contraception in women characterized in that the contraceptive, although of short action, is administered once a month only.

### Technical background of the invention:

Hormonal contraception is considered the most reliable method of reversible contraception today. It requires the continuous taking of pills, generally daily. The pill must be taken every day preferably at the same hour. The vast majority of oral contraceptives consist of a combination of a progestin and estrogen that are administered concurrently for 21 days followed either by a 7 day pill free interval or by the administration of a placebo for 7 days in each 28 day cycle. However the daily intake of pill is constraining for women who often forget it and a bad compliance may have dramatic consequences, including an undesired pregnancy.

Combination oral contraceptives have traditionally acted by suppression of gonadotropins. In addition, it appears that the progestin component is primarily responsible for contraceptive efficacy through inhibition of ovulation, and other peripheral effects which include changes in the cervical mucus (which increase the difficulty of sperm entry into the uterus) and the endometrium (which reduce the likelihood of implantation). However estrogens and progestins are not devoid of adverse effects, including changes in vaginal bleeding, dysmenorrhea, and cardiovascular effects, gastrointestinal disturbances, nausea, weight gain, etc.

Once-a-month injections of hormonal contraceptives have been described.

US patent application 2001/0006963 describes a once-a-month injection with a combination of an estrogenic component and a gestagenic component, each with a sufficiently long action to achieve a contraceptive effect for a one-month period..

US patent 4,780,460 describes a once-a-month intramuscular injection of a glycoester of estradiol which can be co-administered with a long-acting gestagen.

However these methods are still unsatisfactory, as they involve high dosages of estrogens which are associated with side effects, and long action of progestogens.

### Summary of the invention

The invention now provides a method for providing contraception in a woman, wherein the woman is administered with a progestogen-only contraception composition once-a-month between the 1 st and the 10th day of the woman menstrual cycle.

This method meets a long-felt need for women in child-bearing age.

In a preferred embodiment, the progestogen is levonorgestrel.

Preferably, the contraceptive composition is administered orally. In a particular embodiment, the contraceptive composition comprises 1.5mg levonorgestrel for oral administration.

Another subject of the invention is a system used as an aid for reminding women to take the contraceptive composition. An example of such system is an electronic timer device which produces a sound or a light signal once-a-month, on a predetermined point of time in the woman menstrual cycle.

### Brief description of the drawings

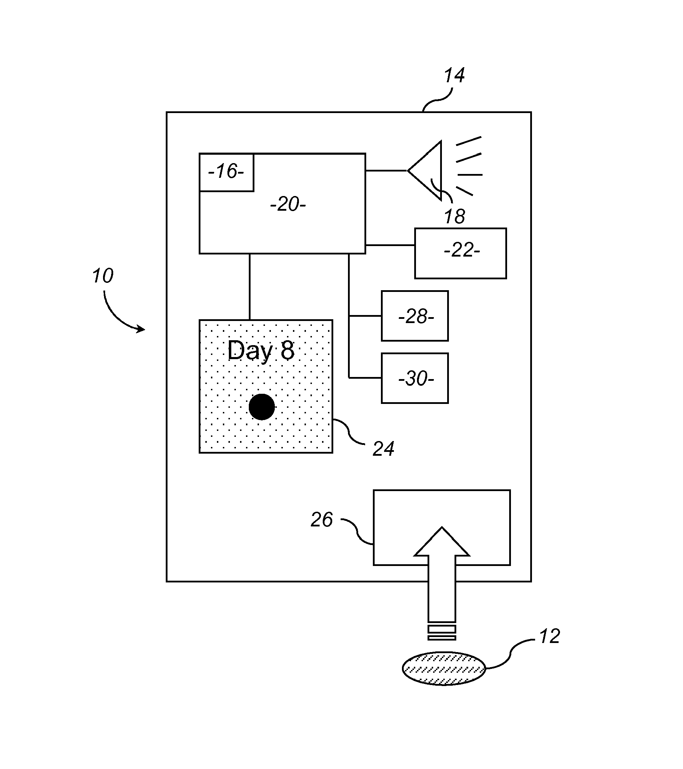

The invention will be better understood with the aid of the description which follows, given solely by way of example while referring to the appended drawing in which the Figure is a schematic representation of a system for reminding a woman of taking a contraceptive composition according to an embodiment of the invention.

### Detailed description of the invention

The inventors have shown that levonorgestrel administered to women at an early stage of the menstrual cycle blocks the development of the ovocyte and consequently prevents ovulation.

On this basis, the inventors propose to use progestogens as regular hormonal contraceptives to be taken once-a-month, rather than every day as usual hormonal contraceptive pills.

According to the invention, a progestogen-only composition is administered in a woman, between the 1^{st} and the 10^{th} day of the woman menstrual cycle, preferably between the 4^{th} and the 10^{th} day of the woman menstrual cycle and more preferably between the 6^{th} and the 10^{th} day.

### Progestogen-only composition

The contraceptive composition is a "progestogen-only" contraceptive, which means that that the progestogen agent is not combined with non-progestogen contraceptive agent, such as an estrogen. In a preferred embodiment, the progestogen is the only active ingredient in the composition.

The progestogen of interest may be selected from progesterone and its derivatives such as, for example, 17-hydroxy progesterone esters, 19-nor-17-hydroxy progesterone esters, 17α-ethinyltestosterone and derivatives thereof, 17α-ethinyl-19-nor-testosterone and derivatives thereof, norethindrone, norethindrone acetate, ethynodiol diacetate, dydrogesterone, medroxy-progesterone acetate, norethynodrel, allylestrenol, lynoestrenol, fuingestanol acetate, medrogestone, norgestrienone, dimethiderome, ethisterone, cyproterone acetate, levonorgestrel, DL-norgestrel, D-17α-acetoxy-13β-ethyl-17α-ethinyl-gon-4-en-3-one oxime, gestodene, desogestrel, norgestimate, nestorone, norethisterone, trimegestone, chlormadione, desogestrel, medrogestone, promegestone, etonogestrel and drospirenone, used alone or in combination with one another.

In a preferred embodiment, the progestogen agent is levonorgestrel.

Levonorgestrel is D-(-)-ethyl- 13β ethynyl-17a hydroxy-17 gonene-4 one-3.

### Once-a-month administration

The subject can be any human female of child bearing-age in need of regular contraception. Generally the women may be between about 12 and about 50 years old. The invention provides a "once-a-month contraception", which means that the woman has to take *(i.e* be administered with) the progestogen-only composition on a single occasion every month, more precisely on a single occasion during each menstrual cycle. In a preferred embodiment, the woman has a regular menstrual cycle, e.g. a 28, 29, 30, or 31 day cycle.

The woman has to take the progestogen-only composition during the late follicular phase of the menstrual cycle. During the late follicular phase, estradiol level increases due to previous stimulation by Follicle Stimulating Hormone (FSH) at the level of ovary. This increased level of estrogen stimulates production of gonadotropin-releasing hormone (GnRH), which increases production of Luteinizing Hormon (LH). LH induces androgen synthesis by thecal cells, stimulates proliferation, differentiation, and secretion of follicular thecal cells and also increases LH receptor expression on granulosa cells. Two or three days after LH level begins to increase, one of the preceding recruited follicles, which contains an ovocyte, has emerged as dominant. The LH rapid surge is normally followed by ovulation. The high estrogen level also initiates the formation of a new layer of endometrium in the uterus.

The progestogen-only composition administered on a single dose every month, during that particular period of time (from the 1st to the 10^{th} day of the menstrual cycle) blocks the development of the ovocyte.

According to the invention, the woman is administered with the single dose of the progestogen-only composition between the 1st (inclusive) and the 10^{th} day (inclusive) of the woman menstrual cycle, preferably between the 4^{th} (inclusive) and the 10^{th} day (inclusive), and more preferably between the 6^{th} (inclusive) and the 10^{th} day (inclusive). The 1st day of the women menstrual cycle is the first day of her period.

### Routes of administration

The progestogen agent may be administered by any convenient route, including oral, buccal, sublingual, parenteral, in particular intramuscular, transdermal, vaginal, in particular in form of a gel, gingival, nasal, rectal, etc.

For a brief review of present methods for drug delivery, see, Langer, Science 249:1527-1533 (1990), which is incorporated herein by reference. Methods for preparing administrable compounds are known or are apparent to those skilled in the art and are described in more detail in, for example, Remington's Pharmaceutical Science, 17th Ed., Mack Publishing Company, Easton, Pa. (1985), which is incorporated herein by reference, and which is hereinafter referred to as "Remington."

According to the invention, the progestogen-only contraceptive is an immediate-release formulation. The contraceptive composition of the invention does not contain long-acting hormone. Its action is based on the rapid effect of a progestogen agent before the ovulation.

The oral route is preferred.

Oral dosage forms are preferentially compressed tablets or capsules. Procedures for the production and manufacture of compressed tablets are well known by those skilled in the art (See Remington). Capsules are solid dosage forms using preferentially either a hard or soft gelatin shell as a container for the mixture of the progestogen agent and inert ingredients. Procedures for production and manufacture of hard gelatin and soft elastic capsules are well known in the art (See Remington).

### Dosage

The unit dosage of progestogen agent *e.g* levonorgestrel to be administered once-a-month may be comprised between 0.10 ± 0.2 mg and 3 ± 0.2 mg, preferably between 0.75 ± 0.2 mg and 2.50 ± 0.2 mg, preferably between 1.50 ± 0.2 mg and 2 ± 0.2 mg, preferably 1.50 ± 0.2 mg.

The progestogen of the invention is preferably in form of an oral dosage, such as a tablet or a capsule, preferably a tablet.

According to preferred embodiment, the composition, preferably in form of a tablet, comprises between 0.10 ± 0.2 mg and 3 ± 0.2 mg levonorgestrel, preferably between 0.75 ± 0.2 mg and 2.50 ± 0.2 mg, preferably between 1.50 ± 0.2 mg and 2 ± 0.2 mg, preferably 1.50 ± 0.2 mg.

The dosages in progestogen agent are superior for an obese women.

In the case of an obese woman, the unit dosage of levonorgestrel is above 1,5 mg, e.g. it is comprised between 1.5mg and 3mg.

According to the invention, an obese woman is used in its most general sense but should be considered relative to the standard criteria for determining obesity.

Obesity is defined as a condition of abnormal or excessive accumulation of adipose tissue, to the extent that health may be impaired. The body mass index (BMI; kg/m²) provides the most useful, albeit crude, population-level measure of obesity. Obesity has also been defined using the WHO classification of the BMI classes for adults: underweight (<18.5), normal weight (18.5 to 24.99), overweight (25 to 29.99), obese grade I (30 to 34.99), obese grade II (35 to 39.99), obese grade III and more (≥40). See WHO, Global database on Body Mass Index, htlp://apps.who.int/index.jsp?introPage=intor 3/html.

Overweight and to obese grade III and more (BMI>25) women are administered with a single dosage of progestogen at a dose superior to women with a BMI below 25.

### Electronic device

It is further provided a system for reminding a woman of taking a contraceptive composition, comprising means for producing an alarm at the expiration of a preprogrammed response time and a contraceptive composition, wherein said contraceptive composition is a progestogen-only composition to be administered once-a-month between the 1st and the 10th day of the woman menstrual cycle, and wherein said means for producing an alarm comprise a timer, wherein which timer is to be activated at a predetermined point of time of the woman menstrual cycle and is to activate said alarm at least once during the period from the 1st to the 10th day of the woman menstrual cycle.

The alarm may be a signal, for instance a sound or a light signal, or it may be a message written on a screen or sent to a predetermined electronic address in an email.

The system may further comprise an automatic preprogrammed actuator for periodically activating said timer at said predetermined point of time of the woman menstrual cycle.

The system may further comprise an actuator to be controlled by said woman for activating said timer at said predetermined point of time of the woman menstrual cycle and/or for deactivating said timer once she has taken said contraceptive composition.

In a particular embodiment, said means for producing an alarm of the system may comprise a portable device with a housing containing :
- a power supply,
- timed alarm means for producing a signal,
- a control unit having an electronic timer device for activating said timed alarm means at the expiration of said preprogrammed response time, and
- a receptacle for enclosing said contraceptive composition.

The unique figure schematically shows a system for reminding a woman of taking a contraceptive composition, according to a particular embodiment of the invention. It comprises means 10, for instance a portable device, for producing an alarm at the expiration of a preprogrammed response time and a contraceptive composition 12, for instance a tablet or a capsule.

Said means 10 comprise a housing 14, a power supply 16, timed alarm means 18 for producing a signal (for instance a sound), a control unit 20 having an electronic timer device 22 for activating said timed alarm means 18 at the expiration of a response time, indicating means 24 on the housing 14 which are connected to the control unit 20 in order to show information, and a receptacle 26 for enclosing the contraceptive composition 12.

The control unit 20 may be preprogrammed and/or programmable by the user to activate the electronic timer device 22 at a predetermined point of time of the menstrual cycle of the user. Said predetermined point of time may be repeatedly defined as the first day of each menstrual cycle, the duration of which (28, 29 ... days) may also be preprogrammed and/or programmable by the user.

The response time of the electronic timer device 22 may also be preprogrammed and/or programmable by the user from one to ten days after the activation of the electronic timer device 22.

As a consequence, the system can be considered as comprising an automatic preprogrammed actuator for periodically activating said timer at a predetermined point of time of the woman menstrual cycle.

Said means 10 may further comprise a first actuator 28, for instance an operating organ on the housing 14 which is connected to the control unit 20, to be controlled by the user for activating manually the electronic timer device 22 in case the periodicity of menstrual cycle would not be as regular as preprogrammed by the automatic actuator. The manual activation may then be considered a priority over the automatic activation.

Finally, said means 10 may further comprise a second actuator 30, for instance an operating organ on the housing 14 which is connected to the control unit 20, to be controlled by the user for deactivating manually the electronic timer device 22 once she has taken said contraceptive composition 12.

In a preferred embodiment of the invention, the control unit 20 and said indicating means 24 are arranged for graphically displaying the days of the woman menstrual cycle and in such a manner that intake of the contraceptive composition (e.g. the pill) is indicated by a flashing point.

In another embodiment (not illustrated), said means 10 for producing an alarm at the expiration of a preprogrammed response time may be embedded in a remote server and may be programmable by the user via Internet through a web page interface for producing said alarm. In that case, the alarm may be a message sent by email or by any other telecommunications channel.

The following example is provided by way of illustration only and not by way of limitation.

### Example: Effect of one intake of levonorgestrel within different stages of the menstrual cycle on women pregnancies.

1,117 women (aged 24.9 +/- 6.5 years) received levonorgestrel (1,5 mg) in form of a single oral tablet during different stages of their menstrual cycle.

Results are presented in Table 1. The reference (day 0) is the ovulation. The fertile period extends from five days before the ovulation to one day after the ovulation.

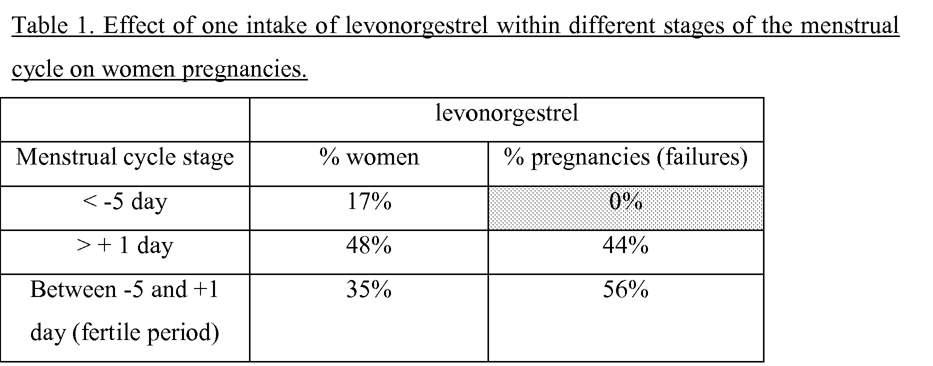

No pregnancy was observed for women who have taken levonorgestrel at least five days before ovulation.

## Claims

1. A method for providing contraception in a woman, wherein the woman is administered with a progestogen-only contraception composition once-a-month between the 1^{st} and the 10^{th} day of the woman menstrual cycle.

2. The method of claim 1, wherein the composition is administered between the 4^{th} and the 10^{th} day of the woman menstrual cycle.

3. The method of the preceding claims, wherein the composition is administered between the 6^{th} and the 10^{th} day of the woman menstrual cycle.

4. The method of anyone of the preceding claims, wherein the progestogen is selected from the group consisting in 17-hydroxy progesterone esters, 19-nor-17-hydroxy progesterone esters, 17α-ethinyltestosterone and derivatives thereof, 17α-ethinyl-19-nor-testosterone and derivatives thereof, norethindrone, norethindrone acetate, ethynodiol diacetate, dydrogesterone, medroxy-progesterone acetate, norethynodrel, allylestrenol, lynoestrenol, fuingestanol acetate, medrogestone, norgestrienone, dimethiderome, ethisterone, cyproterone acetate, levonorgestrel, DL-norgestrel, D-17α-acetoxy-13β-ethyl-17α-ethinyl-gon-4-en-3-one oxime, gestodene, desogestrel, norgestimate, nestorone, norethisterone, trimegestone, chlormadione, desogestrel, medrogestone, promegestone, etonogestrel and drospirenone.

5. The method of anyone of the preceding claims, wherein the progestogen is levonorgestrel.

6. The method of any one of the preceding claims wherein the composition is administered at a dosage of between 0.10 ± 0.2 mg and 3 ± 0.2 mg of progestogen.

7. The method of any one of the preceding claims, wherein the composition is administered at a dosage of 1.50 ± 0.2 mg of progestogen.

8. The method of anyone of the preceding claims, wherein the composition is administered by oral route

9. The method of claim 8, wherein the composition is administered in form of a tablet.

10. A system for reminding a woman of taking a contraceptive composition, comprising means for producing an alarm at the expiration of a preprogrammed response time and a contraceptive composition, wherein said contraceptive composition is a progestogen-only composition to be administered once-a-month between the 1st and the 10th day of the woman menstrual cycle, and in that said means for producing an alarm comprise a timer to be activated at a predetermined point of time of the woman menstrual cycle and to activate said alarm at least once during the period from the 1st to the 10th day of the woman menstrual cycle.

11. The system of claim 10, further comprising an automatic preprogrammed actuator for periodically activating said timer at said predetermined point of time of the woman menstrual cycle.

12. The system of claim 10 or 11, further comprising at least one actuator to be controlled by said woman for activating said timer at said predetermined point of time of the woman menstrual cycle and/or for deactivating said timer once she has taken said contraceptive composition.

13. The system of anyone of claims 10 to 12, wherein said means for producing an alarm comprise a portable device with a housing containing :
- a power supply,
- timed alarm means for producing a signal,
- a control unit having an electronic timer device for activating said timed alarm means at the expiration of said preprogrammed response time, and
- a receptacle for enclosing said contraceptive composition.
